# EUROPÄISCHE PATENTANMELDUNG

(11) **EP 4 255 000 A1**
(43) Veröffentlichungstag der Anmeldung: **04.10.2023**
(21) Anmeldenummer: 23162000.6
(22) Anmeldetag: 15.03.2023
(51) Int. Cl.: H04W 4/80, H04W 4/30, A61B 17/00, H04W 12/47, H04W 12/50, A61B 18/12, H04W 76/10, H04L 9/40, H04W 84/12

(54) **CHIRURGISCHES GERÄTESYSTEM, VERFAHREN ZUM BETRIEB DESSELBEN**

(30) Priorität: 28.03.2022 US 202263324228 P
(71) Anmelder: Olympus Winter & Ibe GmbH, 22045 Hamburg (DE)
(72) Erfinder: Dietrich, Stefan, 14469 Potsdam (DE); Köhnecke, Felix, 22045 Hamburg (DE)
(74) Vertreter: Noack, Andreas

(57) **Zusammenfassung**

Hierin wird ein chirurgisches Vorrichtungssystem bereitgestellt, das Folgendes umfasst: mindestens ein chirurgisches Instrument (11), einen chirurgischen Generator (10) zum Bereitstellen von chirurgischer Energie für mindestens ein chirurgisches Instrument (11), und mindestens eine zusätzliche Vorrichtung (15, 25, 26) zum Bereitstellen einer zusätzlichen Funktion für mindestens einen von dem chirurgischen Generator (10) und/oder dem mindestens einen chirurgischen Instrument (11); der chirurgische Generator (10) so eingerichtet ist, dass er drahtlos mit der mindestens einen Zusatzvorrichtung (15, 25, 26) kommuniziert, wobei der chirurgische Generator (10) und die mindestens eine Zusatzvorrichtung (15, 25, 26) so eingerichtet sind, dass sie einen ersten Kommunikationskanal zum Austausch von Betriebsdaten einrichten, wobei die erste Kommunikation durch eine Anzahl von ersten Kommunikationskanalparametern definiert ist; und wobei der chirurgische Generator (10) und die mindestens eine Zusatzvorrichtung (15, 25, 26) so eingerichtet sind, dass sie einen zweiten Kommunikationskanal zum Austausch von ersten Kommunikationskanalparametern einrichten. Ferner sind Verfahren zum Betrieb eines chirurgischen Gerätesystems vorgesehen.

## Beschreibung

### Gebiet der Erfindung

Die vorliegende Offenbarung bezieht sich auf medizinische Gerätesysteme. Genauer gesagt, bezieht sich die Offenbarung auf die Kommunikation zwischen Zubehörteilen oder Komponenten von medizinischen Gerätesystemen.

### Hintergrund

Die Art und Weise, wie eine Operation durchgeführt wird, hat sich im Laufe der Zeit weiterentwickelt. Während in der klassischen Chirurgie eigenständige mechanische Instrumente wie Skalpelle, Pinzetten, Scheren und dergleichen für verschiedene invasive und nicht-invasive Verfahren verwendet wurden, wurde die mechanische Funktion dieser Instrumente durch Energiefunktionen wie monopolare oder bipolare Elektrochirurgie, Ultraschallchirurgie, Mikrowellenchirurgie und dergleichen ersetzt oder ergänzt. Es wurden chirurgische Generatoren entwickelt, die den jeweiligen Instrumenten chirurgische Energie, meist in Form von elektrischem Strom, zuführen.

Zusätzlich zu den chirurgischen Generatoren wurden weitere Zusatzgeräte entwickelt, die den Betrieb der chirurgischen Generatoren und Instrumente unterstützen. Zu diesen Zusatzgeräten gehören unter anderem Fußschalter, Spülpumpen, Absaugungen, Kühlmittelpumpen und dergleichen.

In einem üblichen Aufbau bilden ein oder mehrere chirurgische Generatoren, ein oder mehrere chirurgische Instrumente und ein oder mehrere Zusatzgeräte ein chirurgisches System zur koordinierten Bereitstellung von chirurgischen und zusätzlichen Funktionen.

Für die notwendige Kommunikation von Betriebsdaten zwischen den Komponenten eines chirurgischen Systems werden die Komponenten häufig über eine Verkabelung verbunden. Eine solche Verkabelung hat jedoch erhebliche Nachteile in Bezug auf die Bedienbarkeit und Manövrierbarkeit im Operationssaal. Um diese Nachteile zu überwinden, wurde in einigen Fällen die drahtlose Kommunikation eingesetzt.

Die sichere drahtlose Kommunikation von Betriebsdaten zwischen Komponenten chirurgischer Systeme erfordert die Verwendung kodierter Kommunikationskanäle. In einigen chirurgischen Gerätesystemen wurde dies dadurch erreicht, dass ein zusätzliches Gerät zur Verwendung mit einem chirurgischen System zusammen mit einer drahtlosen Kommunikationsschnittstelle bereitgestellt wurde, die über eine standardmäßige verdrahtete Schnittstelle wie eine USB-Schnittstelle, eine RS-232-Schnittstelle oder eine proprietäre verdrahtete Schnittstelle an einen chirurgischen Generator angeschlossen werden kann. Die Kommunikationskanalparameter des vom jeweiligen Zusatzgerät verwendeten drahtlosen Kommunikationskanals sind im Zusatzgerät und in der drahtlosen Kommunikationsschnittstelle vorprogrammiert. Beispiele für solche Zusatzgeräte sind die chirurgischen Fußschalter WA91320W und WA94934W, erhältlich bei Olympus Europa SE & Co. KG, Deutschland.

Ein Ziel der vorliegenden Offenbarung ist es, ein chirurgisches System mit verbesserter Flexibilität bereitzustellen.

Einige medizinische Geräte sind so eingerichtet, dass sie eine Vielzahl von Funktionalitäten bieten, wobei einige Funktionalitäten einer separaten Lizenz des Anbieters des medizinischen Geräts unterliegen können. Um solche Funktionen zu aktivieren, kann es erforderlich sein, dass ein Benutzer einen Lizenzschlüssel über eine Benutzerschnittstelle des Medizinprodukts eingeben muss.

Ein weiteres Ziel der vorliegenden Offenbarung ist es, medizinische Geräte bereitzustellen, bei denen die Registrierung von Lizenzen verbessert wird.

### Zusammenfassung der Offenbarung

Die vorliegende Offenbarung stellt ein chirurgisches Vorrichtungssystem bereit, das Folgendes umfasst: mindestens ein chirurgisches Instrument, einen chirurgischen Generator zum Bereitstellen von chirurgischer Energie für mindestens ein chirurgisches Instrument, und mindestens eine Zusatzvorrichtung zum Bereitstellen einer Zusatzfunktion für mindestens einen der beiden chirurgischen Generatoren und/oder das mindestens eine chirurgische Instrument; der chirurgische Generator so eingerichtet ist, dass er drahtlos mit der mindestens einen Zusatzvorrichtung kommuniziert, wobei der chirurgische Generator und die mindestens eine Zusatzvorrichtung so eingerichtet sind, dass sie einen ersten Kommunikationskanal zum Austausch von Betriebsdaten einrichten, wobei die erste Kommunikation durch eine Anzahl von ersten Kommunikationskanalparametern definiert ist; und wobei der chirurgische Generator und die mindestens eine Zusatzvorrichtung so eingerichtet sind, dass sie einen zweiten Kommunikationskanal zum Austausch von ersten Kommunikationskanalparametern einrichten.

Der zweite Kommunikationskanal kann unabhängig von dem ersten Kommunikationskanal sein. Eine Reichweite des zweiten Kommunikationskanals kann kürzer sein als eine Reichweite des ersten Kommunikationskanals. Die Bandbreite des zweiten Kommunikationskanals kann geringer sein als die Bandbreite des ersten Kommunikationskanals. Der zweite Kommunikationskanal kann ein Near Field Communication "NFC"-Kanal sein. Der zweite Kommunikationskanal kann ein Radio Frequency Identification "RFID"-Kanal sein.

Der erste Kommunikationskanal kann ein IEEE 802.11 "WiFi" Kommunikationskanal sein. Bei dem ersten Kommunikationskanal kann es sich um einen "Bluetooth"-Kommunikationskanal handeln. Der Begriff "Bluetooth" bezieht sich hier auf alle verfügbaren Versionen des "Bluetooth"-Kommunikationsstandards, der von der Bluetooth Special Interest Group bereitgestellt wird. Bei dem ersten Kommunikationskanal kann es sich um einen IEEE802.15.4- oder "ZigBee"-Kommunikationskanal handeln.

Das mindestens eine Zusatzgerät kann ferner so eingerichtet sein, dass es Gerätecharakterisierungsdaten und/oder Geräteidentifikationsdaten über den zweiten Kommunikationskanal überträgt. Das mindestens eine zusätzliche Gerät kann mindestens einen Fußschalter, einen Rauchabzug, eine Spülpumpe, eine Absaugung oder eine Kühlmittelpumpe umfassen. Die Betriebsdaten umfassen mindestens eines der folgenden Signale: ein Aktivierungssignal, ein Deaktivierungssignal, eine Zielflussrate, eine aktuelle Flussrate, einen Zieldruck, einen aktuellen Druck, eine Zieltemperatur, eine aktuelle Temperatur, einen Batteriestatus, eine Verbindungsqualität und ein Herzschlagsignal.

Der chirurgische Generator kann ferner so eingerichtet sein, dass er über eine Netzwerkverbindung Software-Aktualisierungsdaten für eine oder mehrere der zusätzlichen Vorrichtungen empfängt und solche Software-Aktualisierungsdaten an die eine oder mehrere zusätzliche Vorrichtungen übermittelt.

Die vorliegende Offenbarung stellt ferner ein chirurgisches Vorrichtungssystem zur Verfügung, das mindestens ein chirurgisches Instrument, einen chirurgischen Generator zur Bereitstellung chirurgischer Energie für mindestens ein chirurgisches Instrument und mindestens eine Zusatzvorrichtung zur Bereitstellung einer Zusatzfunktion für mindestens den chirurgischen Generator und/oder das mindestens eine chirurgische Instrument umfasst; wobei der chirurgische Generator so eingerichtet ist, dass er mit der mindestens einen Zusatzvorrichtung kommuniziert; wobei der chirurgische Generator und die mindestens eine Zusatzvorrichtung so eingerichtet sind, dass sie einen ersten, drahtgebundenen Kommunikationskanal zum Austausch von Betriebsdaten aufbauen; und wobei der chirurgische Generator und die mindestens eine Zusatzvorrichtung so eingerichtet sind, dass sie einen zweiten, drahtlosen Kommunikationskanal zum Austausch von Zusatzdaten aufbauen.

Die vorliegende Offenbarung stellt einen chirurgischen Generator eines chirurgischen Gerätesystems gemäß der obigen Offenbarung bereit.

Die vorliegende Offenbarung stellt eine zusätzliche Vorrichtung eines chirurgischen Gerätesystems gemäß der obigen Offenbarung bereit.

Die vorliegende Offenbarung stellt ferner ein Verfahren zum Betreiben eines chirurgischen Gerätesystems bereit, mit den Schritten: Einrichten eines zweiten Kommunikationskanals zwischen einem chirurgischen Generator und einem Zusatzgerät, Austauschen von ersten Kommunikationskanalparametern über den zweiten Kommunikationskanal, Einrichten eines ersten Kommunikationskanals zwischen dem chirurgischen Generator und dem Zusatzgerät unter Verwendung der ersten Kommunikationskanalparameter, und Austauschen von Betriebsdaten zwischen dem chirurgischen Generator und dem mindestens einen Zusatzgerät über den ersten Kommunikationskanal.

Ein Verfahren gemäß der vorliegenden Offenbarung kann ferner einen Schritt der Übertragung von Gerätecharakterisierungsdaten und/oder Geräteidentifikationsdaten des mindestens einen Zusatzgeräts über den zweiten Kommunikationskanal umfassen.

Die vorliegende Offenbarung stellt ferner ein Verfahren zum Betreiben eines chirurgischen Gerätesystems bereit, das die folgenden Schritte umfasst Empfangen von Eingabedaten, die einen durchzuführenden chirurgischen Eingriff identifizieren, über eine Benutzerschnittstelle; Auslesen einer Liste von Zusatzgeräten, die für den durchzuführenden chirurgischen Eingriff erforderlich sind, aus einem Speicher; Aufbauen eines zweiten Kommunikationskanals zwischen dem elektrochirurgischen Generator und einem oder mehreren Zusatzgeräten innerhalb der Reichweite des zweiten Kommunikationskanals; Empfangen von Geräteidentifikationsdaten und/oder Gerätecharakterisierungsdaten von zusätzlichen Geräten innerhalb der Reichweite des zweiten Kommunikationskanals über den zweiten Kommunikationskanal; Prüfen, ob alle zusätzlichen Geräte, die für das durchzuführende Verfahren erforderlich sind, Geräteidentifikationsdaten oder Gerätecharakterisierungsdaten als Antwort auf das Abfragesignal bereitgestellt haben; und, wenn die Prüfung erfolgreich war, Aufbauen eines oder mehrerer erster Kommunikationskanäle zwischen dem chirurgischen Generator und den zusätzlichen Geräten.

Das Verfahren kann ferner einen Schritt umfassen, bei dem, wenn die Prüfung nicht erfolgreich war, über die Benutzerschnittstelle eine Fehlermeldung ausgegeben wird, die fehlende Zusatzgeräte identifiziert, die für den durchzuführenden Eingriff erforderlich sind.

Die vorliegende Offenbarung stellt ferner ein medizinisches Gerät bereit, das eine Steuereinheit und eine Funktionseinheit umfasst, wobei das medizinische Gerät so konfiguriert ist, dass es eine Vielzahl von medizinischen Funktionalitäten über die Funktionseinheit bereitstellt, wobei mindestens eine der Vielzahl von Funktionalitäten einer Lizenz unterliegt, wobei das medizinische Gerät ferner eine drahtlose Kommunikationseinheit umfasst, die so konfiguriert ist, dass sie einen drahtlosen Kommunikationskanal mit einem drahtlosen Lizenztag einrichtet und einen Lizenzschlüssel für die mindestens eine Funktionalität von dem drahtlosen Lizenztag erhält. Das medizinische Gerät kann so eingerichtet sein, dass es nach dem Aufbau des drahtlosen Kommunikationskanals mit dem drahtlosen Lizenztag Identifikationsdaten des medizinischen Geräts über den drahtlosen Kommunikationskanal übermittelt.

Die vorliegende Offenbarung stellt ferner ein drahtloses Lizenztag bereit, wobei das drahtlose Lizenztag so eingerichtet ist, dass es einen drahtlosen Kommunikationskanal mit einem medizinischen Gerät einrichtet und einen Lizenzschlüssel an das medizinische Gerät übermittelt, wobei der Lizenzschlüssel in einem Speicherelement des drahtlosen Lizenztags gespeichert ist. Der drahtlose Lizenztag kann so eingerichtet sein, dass er den Lizenzschlüssel in einem ersten Zustand und einem zweiten Zustand speichert, wobei der erste Zustand einen unbenutzten Lizenzschlüssel darstellt und der zweite Zustand einen benutzten Lizenzschlüssel darstellt. Der drahtlose Lizenztag kann so eingerichtet sein, dass er den Zustand des gespeicherten Lizenzschlüssels nach der Übertragung des Lizenzschlüssels an das medizinische Gerät vom ersten Zustand in den zweiten Zustand ändert.

Der drahtlose Lizenztag kann so eingerichtet sein, dass er nach dem Aufbau des drahtlosen Kommunikationskanals mit dem medizinischen Gerät Identifikationsdaten des medizinischen Geräts über den drahtlosen Kommunikationskanal empfängt und die Identifikationsdaten des medizinischen Geräts zusammen mit dem Lizenzschlüssel in dem zweiten Zustand speichert. Der drahtlose Lizenztag kann so eingerichtet sein, dass er, wenn der Lizenzschlüssel im zweiten Zustand gespeichert ist, den Lizenzschlüssel nur dann an das medizinische Gerät übermittelt, wenn die vom medizinischen Gerät empfangenen Identifikationsdaten des medizinischen Geräts mit den mit dem Lizenzschlüssel gespeicherten Identifikationsdaten des medizinischen Geräts übereinstimmen.

Einige Beispiele der vorliegenden Offenbarung werden im Folgenden anhand von illustrativen Zeichnungen beschrieben. Die beschriebenen Beispiele dienen dem besseren Verständnis und sollen den Umfang der beigefügten Ansprüche in keiner Weise einschränken.

### Kurze Beschreibung der Zeichnungen

Die Zeichnungen zeigen:
Abb. 1: Ein chirurgisches Gerätesystem,
Fig. 2: einen elektrochirurgischen Generator in einer schematischen Ansicht,
Fig. 3: eine Zusatzvorrichtung in schematischer Darstellung,
Fig. 4: ein Verfahren zum Betrieb eines chirurgischen Gerätesystems,
Fig. 5: ein weiteres Verfahren zum Betrieb eines chirurgischen Gerätesystems,
Fig. 6: ein chirurgisches Gerät mit modularem Aufbau,
Abb. 7: ein medizinisches Gerätesystem,
Abb. 8: ein Verfahren zum Betrieb eines medizinischen Gerätesystems,
Abb. 9: ein weiteres chirurgisches Gerätesystem.

### Ausführliche Beschreibung

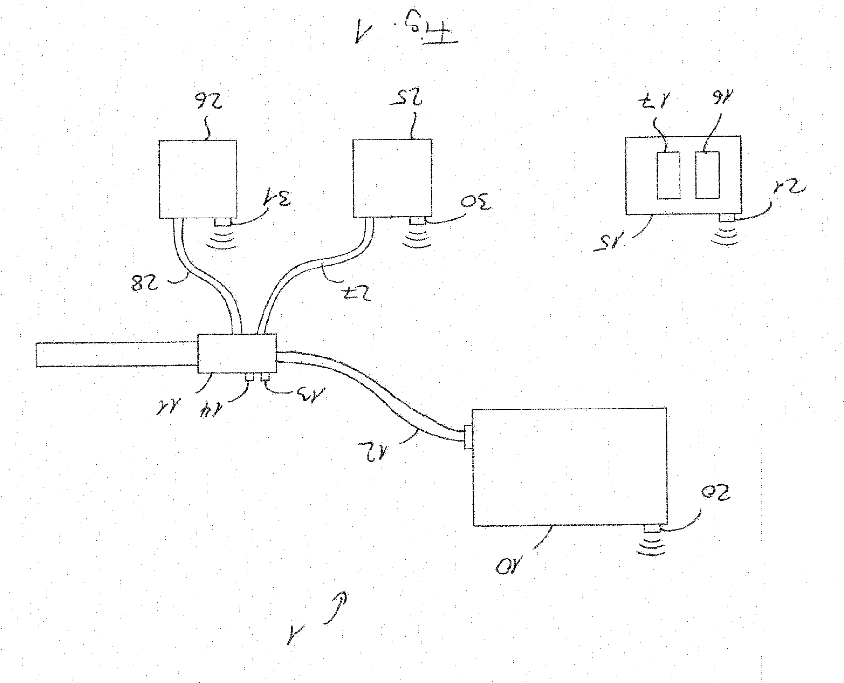

Fig. 1 zeigt ein chirurgisches Gerätesystem 1, das einen elektrochirurgischen Generator 10 und ein elektrochirurgisches Instrument 11 umfasst. Das elektrochirurgische Instrument 11 ist mit dem elektrochirurgischen Generator 10 über ein Kabel 12 zur Übertragung von elektrochirurgischen Therapiesignalen vom elektrochirurgischen Generator 10 zum elektrochirurgischen Instrument 11 und zum Austausch von Konfigurations- und/oder Betriebsdaten zwischen dem elektrochirurgischen Generator 10 und dem elektrochirurgischen Instrument 11 verbunden. Das elektrochirurgische Instrument 11 kann Aktivierungsknöpfe 13, 14 an einem Griffteil des elektrochirurgischen Instruments 11 umfassen, so dass sie von einem Arzt, der das elektrochirurgische Instrument 11 hält, betätigt werden können. Aktivierungssignale von den Aktivierungstasten 13, 14 können über das Kabel 12 an den elektrochirurgischen Generator 10 weitergeleitet werden.

Bei dem elektrochirurgischen Generator 10 kann es sich um einen Radiofrequenzgenerator, einen Mikrowellengenerator, einen Ultraschallgenerator oder eine Kombination davon handeln. Bei dem elektrochirurgischen Instrument 11 kann es sich um ein Schneidinstrument, ein Versiegelungsinstrument, ein Ablationsinstrument oder eine Kombination davon handeln. Das chirurgische Gerätesystem 1 umfasst außerdem einen drahtlosen Fußschalter 15 mit zwei Pedalen 16, 17. Der drahtlose Fußschalter 15 kann mehr oder weniger als zwei Pedale haben und zusätzliche Bedienelemente wie Kippschalter, Schalter oder ähnliches umfassen.

Der elektrochirurgische Generator 10 und der drahtlose Fußschalter 15 umfassen jeweils eine drahtlose Kommunikationseinheit 20, 21. Im vorliegenden Beispiel können die drahtlosen Kommunikationseinheiten 20, 21 so eingerichtet sein, dass sie über elektromagnetische Signale unter Verwendung des IEEE 802.11-Standards, allgemein bekannt als "WiFi", miteinander kommunizieren. Daher verwenden die drahtlosen Kommunikationseinheiten 20, 21 einen ersten drahtlosen Kommunikationskanal, der durch eine Reihe von Kommunikationskanalparametern definiert ist, einschließlich, aber nicht beschränkt auf einen Kanalidentifikationscode, einen Kanalauthentifizierungscode und dergleichen. Alternativ können auch andere elektromagnetische Kommunikationsstandards wie "Bluetooth" oder "ZigBee" für den ersten drahtlosen Kommunikationskanal verwendet werden.

Im vorliegenden Beispiel umfasst das chirurgische Gerätesystem weitere Zusatzgeräte wie eine Spülpumpe 25 und eine Absaugung 26, die jeweils über Röhren 27, 28 mit dem chirurgischen Instrument 11 verbunden sind. Die Spülpumpe 25 kann verwendet werden, um eine Spülflüssigkeit über die Röhre 27 und das elektrochirurgische Instrument 11 zu einer Operationsstelle zu leiten. Die Spülflüssigkeit kann verwendet werden, um Ablagerungen oder Blut von der Stelle wegzuspülen. Die Absaugung 26 kann dazu verwendet werden, Flüssigkeiten, Dämpfe oder Gase über das elektrochirurgische Instrument 11 und die Röhre 28 aus dem Operationsgebiet abzusaugen.

Die Spülpumpe 25 und die Absaugung 26 umfassen auch drahtlose Kommunikationseinheiten 30, 31. Die drahtlosen Kommunikationseinheiten können auch so eingerichtet sein, dass sie untereinander und/oder mit einer oder mehreren der Kommunikationseinheiten 20, 21 durch elektromagnetische Signale kommunizieren.

Während des Betriebs des chirurgischen Gerätesystems 1 kann ein Benutzer eine der Aktivierungstasten 13, 14 und/oder die Pedale 16, 17 des drahtlosen Fußschalters betätigen, um verschiedene Funktionen des chirurgischen Gerätesystems 1 zu aktivieren.

Das erste Pedal 16 kann, wenn es gedrückt wird, die Ausgabe eines ersten elektrochirurgischen Therapiesignals vom elektrochirurgischen Generator 10 an das elektrochirurgische Instrument 11 auslösen. Bei dem ersten elektrochirurgischen Signal kann es sich um ein Schneidesignal handeln, so dass das elektrochirurgische Instrument 11 zum Schneiden durch das betreffende Gewebe verwendet werden kann. Das zweite Pedal kann bei Betätigung die Abgabe eines zweiten elektrochirurgischen Therapiesignals vom elektrochirurgischen Generator 10 an das elektrochirurgische Instrument 11 auslösen. Bei dem zweiten elektrochirurgischen Signal kann es sich um ein Kauterisationssignal handeln, so dass das elektrochirurgische Instrument 11 dazu verwendet werden kann, Blutungen zu kauterisieren, z. B. Blutungen, die von einem früheren Gewebeschnitt herrühren. Die Betätigung eines der Pedale 16, 17 des drahtlosen Fußschalters 15 wird über die drahtlosen Kommunikationseinheiten 21, 20 an den elektrochirurgischen Generator 10 übermittelt.

Die erste Aktivierungstaste 13 am elektrochirurgischen Instrument 11 kann die Spülpumpe 25 aktivieren. Der Arzt kann die Aktivierungstaste 13 z. B. dann drücken, wenn sich Blut oder Ablagerungen im Operationsgebiet ansammeln, so dass eine Flüssigkeit wie sterile Kochsalzlösung aus dem elektrochirurgischen Instrument 11 ausgestoßen werden kann, um das Blut oder die Ablagerungen wegzuspülen. Die zweite Aktivierungstaste 14 am elektrochirurgischen Instrument 11 kann die Absaugung 26 aktivieren. Der Arzt kann die Aktivierungstaste 14 drücken, wenn z. B. Dämpfe, Blut oder Spülflüssigkeit aus dem Operationsgebiet abgesaugt werden müssen. Die Aktivierungssignale der Aktivierungstasten 13, 14 werden über das Kabel 12 an den elektrochirurgischen Generator 10 übermittelt und dann über die drahtlosen Kommunikationseinheiten 20, 30, 31 an die Absaugung 25 und die Spülpumpe 26 weitergeleitet. Für die Kommunikation zwischen den drahtlosen Kommunikationseinheiten 20, 30, 31 kann derselbe Kommunikationskanal verwendet werden wie für die Kommunikation zwischen den drahtlosen Kommunikationseinheiten 20 und 21.

Um die Kommunikation zwischen den Geräten des chirurgischen Gerätesystems 1 wie oben beschrieben zu erleichtern, müssen sich die drahtlosen Kommunikationseinheiten 20, 21, 30, 31 auf Kommunikationskanalparameter einigen. In Systemen nach dem Stand der Technik wurde dies dadurch erreicht, dass jedes Zusatzgerät mit einem eigenen Paar drahtloser Kommunikationseinheiten ausgestattet wurde, von denen eine fest mit dem Zusatzgerät verbunden ist, während die andere über eine kabelgebundene Standardschnittstelle wie einen USB-Stecker oder eine proprietäre kabelgebundene Schnittstelle an den elektrochirurgischen Generator angeschlossen wird. Eine solche Konstruktion ist jedoch bei mehreren Zusatzgeräten tendenziell unhandlich.

In einem System gemäß der vorliegenden Offenbarung vereinbaren die drahtlosen Kommunikationseinheiten 20, 21, 30, 31 die Kommunikationskanalparameter über einen zweiten drahtlosen Kommunikationskanal. Der zweite drahtlose Kommunikationskanal kann zwischen den drahtlosen Kommunikationseinheiten 20, 21, 30, 31 unter Verwendung von RFID- oder NFC-Technologie eingerichtet werden. Daher kann die drahtlose Kommunikationseinheit 20 einen RFID-Abfragesender enthalten, und die drahtlosen Kommunikationseinheiten 21, 30, 31 können RFID-Transponder enthalten. Der zweite Kommunikationskanal wird durch Senden eines Abfragesignals durch den RFID-Abfragesender der drahtlosen Kommunikationseinheit 20 und durch Empfangen des Abfragesignals durch die RFID-Transponder der drahtlosen Kommunikationseinheiten 21, 30, 31 aufgebaut. Das Abfragesignal kann Kommunikationskanalparameter für den ersten Kommunikationskanal enthalten, die in der drahtlosen Kommunikationseinheit 20 vorprogrammiert sind und dann von den drahtlosen Kommunikationseinheiten 21, 30, 31 angewendet werden können. Alternativ dazu können die Kommunikationskanalparameter zwischen den drahtlosen Kommunikationseinheiten 20, 21, 30 und 31 ausgehandelt werden.

Eine verfügbare Kommunikationsreichweite des zweiten drahtlosen Kommunikationskanals, der RFID- oder NFC-Technologie verwendet, ist viel kürzer als eine verfügbare Kommunikationsreichweite des ersten Kommunikationskanals. Daher kann es notwendig sein, die Zusatzgeräte 15, 25, 26 beim Einrichten des Chirurgiegerätesystems in die Nähe des elektrochirurgischen Generators 10 zu bringen, damit der zweite Kommunikationskanal aufgebaut werden kann. Dies hat den technischen Vorteil, dass Schutzmaßnahmen des zweiten Kommunikationskanals gegen unbefugten Zugriff durch andere Geräte nicht notwendig sein müssen. Für den ersten Kommunikationskanal lassen sich solche Schutzmaßnahmen leichter anwenden, da notwendige Parameter zuvor von den beteiligten Geräten über den zweiten Kommunikationskanal vereinbart werden können.

Der zweite Kommunikationskanal kann auch eine geringere Kommunikationsbandbreite haben als der erste Kommunikationskanal. Dies stellt kein Problem dar, da die über den zweiten Kommunikationskanal zu übertragende Datenmenge eher gering ist und nur im Bereich von wenigen Kilobyte liegt.

Nach dem Empfang des Abfragesignals können die drahtlosen Kommunikationseinheiten 21, 30, 31 Antwortsignale senden, die von der drahtlosen Kommunikationseinheit 20 empfangen werden. Die Antwortsignale können Identifikations- und/oder Charakterisierungsinformationen von den Zusatzgeräten 15, 25, 26 enthalten.

Der elektrochirurgische Generator 10 kann die von den Zusatzgeräten 15, 25, 26 empfangenen Identifikations- und/oder Charakterisierungsinformationen verwenden, um zu überprüfen, ob alle für ein beabsichtigtes Verfahren erforderlichen Zusatzgeräte verfügbar sind. Daher kann ein Benutzer über eine Benutzerschnittstelle des elektrochirurgischen Generators 10 ein bestimmtes Verfahren aus einer Liste verfügbarer Verfahren auswählen. Der elektrochirurgische Generator 10 kann dann eine Liste der erforderlichen Zusatzgeräte aus einem Speicher, z. B. einem internen Speicher des elektrochirurgischen Generators 10 oder einem externen Speicher, der mit dem elektrochirurgischen Generator 10 verbunden ist, lesen.

Wenn der elektrochirurgische Generator 10 feststellt, dass alle erforderlichen Zusatzgeräte verfügbar sind, kann der elektrochirurgische Generator 10 damit fortfahren, den ersten drahtlosen Kommunikationskanal mit den Zusatzgeräten 15, 25, 26 aufzubauen, so dass das ausgewählte Verfahren durchgeführt werden kann. Stellt der elektrochirurgische Generator 10 fest, dass eine oder mehrere erforderliche Zusatzeinrichtungen kein Antwortsignal auf das Abfragesignal gesendet haben, kann eine entsprechende Fehlermeldung über eine Benutzerschnittstelle des elektrochirurgischen Generators 10 ausgegeben werden.

Während der Durchführung des gewählten Verfahrens werden Betriebsdaten zwischen dem HF-Generator 10 und den Zusatzgeräten 15, 25, 26 ausgetauscht. Zu den Betriebsdaten können Aktivierungs- oder Deaktivierungssignale gehören, die vom drahtlosen Fußschalter 15 an den elektrochirurgischen Generator 10 oder vom elektrochirurgischen Generator 10 an die Absaugpumpe 25 oder die Spülpumpe 26 gesendet werden. Die Betriebsdaten können ferner Soll- oder Ist-Durchflussraten der Pumpen 25, 26, Soll- oder Ist-Drücke der Pumpen 25, 26 oder Ähnliches umfassen.

In anderen, in den Zeichnungen nicht dargestellten Ausführungsformen eines chirurgischen Gerätesystems kann eine Kühlmittelpumpe als zusätzliches Gerät verwendet werden. In diesem Fall können die Betriebsdaten die Soll- oder Ist-Zufluss- und -Abfluss-Temperaturen eines Kühlmittels umfassen. In weiteren, in den Zeichnungen nicht dargestellten Ausführungsformen eines chirurgischen Gerätesystems kann eine Rauchabsaugung als zusätzliche Einrichtung verwendet werden. In diesem Fall können die Betriebsdaten Ziel- oder aktuelle Evakuierungsvolumenströme umfassen.

Die Betriebsdaten können ferner Daten zum Batteriestatus und/oder zur Verbindungsqualität enthalten. Die Betriebsdaten können außerdem ein oder mehrere Heartbeat-Signale enthalten. Die Heartbeat-Signale können vom elektrochirurgischen Generator 10 und/oder den Zusatzgeräten 15, 25, 26 verwendet werden, um festzustellen, ob der erste drahtlose Kommunikationskanal noch verfügbar ist. Im Falle eines Verbindungsverlustes des ersten drahtlosen Kommunikationskanals können der elektrochirurgische Generator 10 und/oder die zusätzlichen Geräte 15, 25, 26 so eingerichtet werden, dass sie geeignete Maßnahmen zur Aufrechterhaltung der Patientensicherheit ergreifen.

In einer weiteren Ausführungsform eines chirurgischen Gerätesystems kann der elektrochirurgische Generator so eingerichtet sein, dass er über eine Netzwerkverbindung (nicht dargestellt) Software-Aktualisierungsdaten für ein oder mehrere zusätzliche Zielgeräte der zusätzlichen Geräte 15, 25, 26 empfängt. Solche Software-Aktualisierungsdaten können von dem elektrochirurgischen Generator 10 jederzeit empfangen und in einem Speicher des elektrochirurgischen Generators 10 gespeichert werden. Nachdem der erste drahtlose Kommunikationskanal zwischen dem elektrochirurgischen Generator 10 und dem einen oder den mehreren zusätzlichen Zielgeräten eingerichtet wurde, kann der elektrochirurgische Generator 10 die Software-Aktualisierungsdaten an die zusätzlichen Zielgeräte übermitteln, und die zusätzlichen Zielgeräte können die Software-Aktualisierungsdaten zur Aktualisierung ihrer internen Betriebssoftware verwenden.

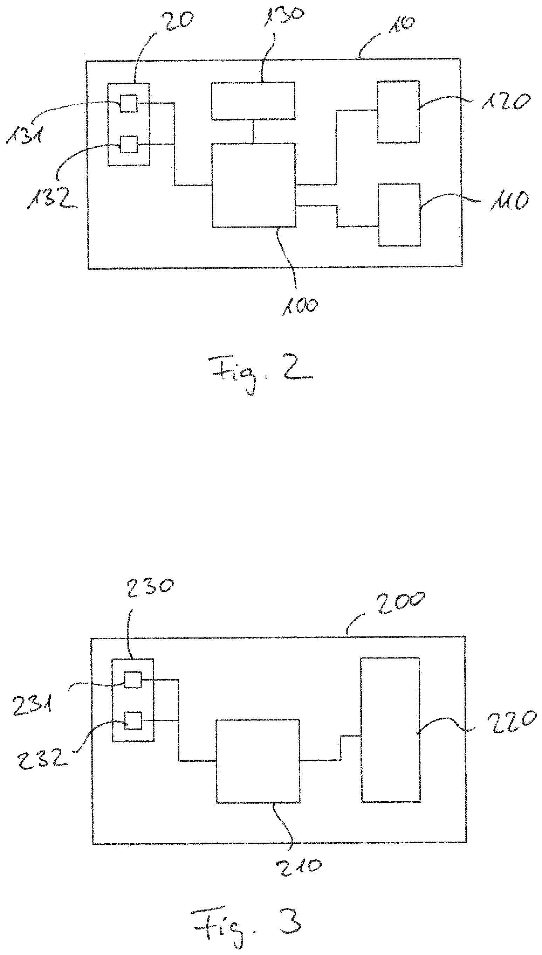

Der interne Aufbau des elektrochirurgischen Generators 10 ist in Fig. 2 schematisch dargestellt. Der elektrochirurgische Generator 10 umfasst eine Steuereinheit 100 zur Steuerung verschiedener Funktionen des elektrochirurgischen Generators 10. Die Steuereinheit 100 kann eine Standard-Computerarchitektur mit einem oder mehreren Prozessoren und einem oder mehreren Speicherelementen aufweisen. Eine solche Standard-Computerarchitektur ist dem Fachmann bekannt und muss hier nicht im Detail erläutert werden.

Der elektrochirurgische Generator 10 umfasst ferner eine elektrochirurgische Funktionseinheit 110. Die elektrochirurgische Funktionseinheit kann einen Radiofrequenzgenerator, einen Ultraschallgenerator, einen Mikrowellengenerator oder eine Kombination davon umfassen. Der Aufbau solcher elektrochirurgischen Funktionseinheiten ist aus dem Stand der Technik bekannt und muss hier nicht im Detail erläutert werden.

Der elektrochirurgische Generator 10 umfasst ferner eine Benutzerschnittstelle 120. Die Benutzerschnittstelle 120 kann eine oder mehrere Anzeigen, Steuertasten, Tastaturen, berührungsempfindliche Anzeigen oder Kombinationen davon umfassen. Über die Benutzerschnittstelle 120 kann die Steuereinheit Betriebs- und Statusinformationen an einen Benutzer übermitteln und Eingaben von einem Benutzer empfangen. Die Eingaben eines Benutzers können unter anderem die Auswahl eines durchzuführenden Verfahrens, die Auswahl von Betriebsparametern für ein ausgewähltes Verfahren oder Ähnliches umfassen. Der elektrochirurgische Generator umfasst ferner eine Speichereinheit 130. Die Speichereinheit 130 kann Teil der Steuereinheit 100 sein oder von dieser getrennt sein. Bei der Speichereinheit 130 kann es sich um einen statischen Speicher mit wahlfreiem Zugriff (S-RAM), einen dynamischen Speicher mit wahlfreiem Zugriff (D-RAM), einen Festwertspeicher (ROM), ein Festplattenlaufwerk (HDD), eine Solid State Disk (SSD) oder eine Kombination davon handeln. Die Speichereinheit 130 kann eine Liste von elektrochirurgischen Verfahren speichern, die mit dem elektrochirurgischen Generator 10 durchgeführt werden können. Für jedes verfügbare Verfahren kann die Speichereinheit eine Liste der für das jeweilige Verfahren erforderlichen Zusatzgeräte speichern.

Der elektrochirurgische Generator umfasst außerdem die drahtlose Kommunikationseinheit 20. Die drahtlose Kommunikationseinheit 20 umfasst eine WiFi-Antenne 131, einen RFID-Abfragesender 132 und zugehörige Schaltungen (nicht dargestellt).

Die Steuereinheit 100 kommuniziert mit der elektrochirurgischen Funktionseinheit 110, der Benutzerschnittstelle 120, der Speichereinheit 10 und der drahtlosen Kommunikationseinheit 20, um wie oben beschrieben zu arbeiten.

Fig. 3 zeigt den inneren Aufbau eines Zusatzgerätes 200 in einer schematischen Darstellung. Die Zusatzeinrichtung 200 kann ein Fußschalter ähnlich dem Fußschalter 15, eine Pumpe ähnlich der Absaugpumpe 25 oder der Spülpumpe 26 oder eine andere Zusatzeinrichtung sein. Die Zusatzeinrichtung 200 umfasst eine Steuereinheit 210 für die Zusatzeinrichtung und eine zusätzliche Funktionseinheit 220. Die Steuereinheit 210 des Zusatzgeräts kann eine Standard-Computerarchitektur aufweisen, die der Steuereinheit 100 des elektrochirurgischen Generators 10 ähnlich ist. Der Aufbau der Zusatzfunktionseinheit kann je nach Art der Zusatzfunktion, die das Zusatzgerät 200 bereitstellen soll, sehr unterschiedlich sein.

Handelt es sich bei der Zusatzeinrichtung 200 um einen Fußschalter wie den Fußschalter 15, so kann die Zusatzfunktionseinheit 220 ein oder mehrere Pedale und einen oder mehrere Sensoren umfassen, die erfassen, ob das eine oder die mehreren Pedale gedrückt sind oder nicht, sowie optional eine auf das eine oder die mehreren Pedale ausgeübte Kraft. Handelt es sich bei der Zusatzvorrichtung 200 um eine Pumpe wie die Absaugpumpe 25, die Bewässerungspumpe 26 oder eine Kühlmittelpumpe, kann die Zusatzfunktion 200 eine Pumpeneinheit wie eine Schlauchpumpe, eine Kolbenpumpe oder dergleichen, eine Antriebseinheit zum Antreiben der Pumpeneinheit und einen oder mehrere Sensoren zum Erfassen eines oder mehrerer Werte aus dem Druck des gepumpten Mediums, der Durchflussmenge des gepumpten Mediums und der Temperatur des gepumpten Mediums umfassen.

Die Zusatzvorrichtung 200 umfasst ferner eine drahtlose Kommunikationseinheit 230, die eine WiFi-Antenne 231 und einen RFID-Transponder 232 umfasst. Die Steuereinheit 210 der Zusatzvorrichtung kommuniziert mit der Zusatzfunktionseinheit 220 und der drahtlosen Kommunikationseinheit 230, um wie oben beschrieben zu arbeiten.

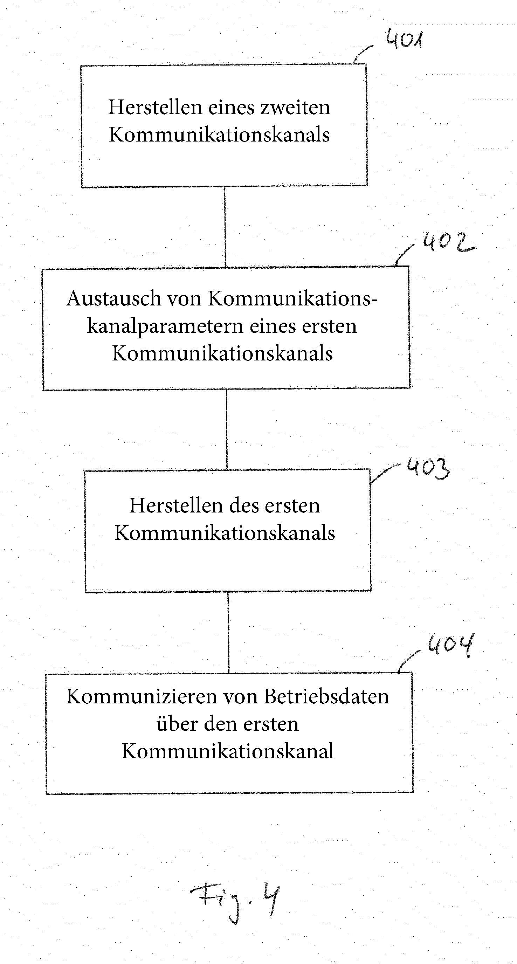

Fig. 4 zeigt ein Verfahren zum Betrieb eines chirurgischen Gerätesystems wie das oben beschriebene. In einem ersten Schritt 401 wird ein zweiter Kommunikationskanal zwischen einem elektrochirurgischen Generator und einem oder mehreren Zusatzgeräten aufgebaut.

Zum Aufbau des zweiten Kommunikationskanals kann der elektrochirurgische Generator ein Abfragesignal über eine drahtlose Kommunikationseinheit senden. Bei dem Abfragesignal kann es sich um ein RFID-Abfragesignal handeln. Das Abfragesignal kann von zusätzlichen Geräten innerhalb der Reichweite des Abfragesignals empfangen werden. Nach dem Empfang des Abfragesignals kann jedes zusätzliche Gerät ein Antwortsignal senden, das von dem elektrochirurgischen Generator empfangen werden kann. Durch diese Abfrage und Antwort wird der zweite Kommunikationskanal zwischen dem elektrochirurgischen Generator und dem einen oder den mehreren zusätzlichen Geräten hergestellt.

In einem zweiten Schritt 402 tauschen der elektrochirurgische Generator und das eine oder die mehreren Zusatzgeräte Kommunikationskanalparameter eines ersten Kommunikationskanals aus, der zwischen dem elektrochirurgischen Generator und dem einen oder den mehreren Zusatzgeräten eingerichtet werden soll. In einigen Ausführungsformen umfassen die Kommunikationskanalparameter vorbestimmte Parameter, die in einem Speicher des elektrochirurgischen Generators gespeichert sind. Solche vorgegebenen Parameter können beispielsweise ein Frequenzband wie 2,4 GHz, 4,9 GHz oder ähnliches und eine Kanalnummer wie einer der Kanäle 1 bis 14 im 2,4-GHz-Band einer WiFi-Verbindung umfassen. In einigen Ausführungsformen können die Parameter des Kommunikationskanals dynamische Parameter enthalten, die zwischen dem elektrochirurgischen Generator und dem einen oder den mehreren zusätzlichen Geräten ausgehandelt oder vereinbart werden. Zu diesen dynamischen Parametern können verschlüsselungsbezogene Parameter gehören, die z. B. durch den Austausch von kryptografischen Schlüsseln zwischen dem elektrochirurgischen Generator und dem einen oder den mehreren Zusatzgeräten bestimmt werden können.

In einer möglichen Ausführungsform können der erste und der zweite oben beschriebene Schritt 401, 402 in einem einzigen Schritt zusammengefasst werden. In diesem Fall können alle erforderlichen Kommunikationskanalparameter in das vom elektrochirurgischen Generator gesendete Abfragesignal aufgenommen werden.

Zusammen mit dem Antwortsignal können das eine oder die mehreren Zusatzgeräte Gerätecharakterisierungsdaten und/oder Geräteidentifikationsdaten an den elektrochirurgischen Generator übertragen. Die Gerätecharakterisierungsdaten und/oder Geräteidentifikationsdaten des mindestens einen zusätzlichen Geräts können vom elektrochirurgischen Generator verwendet werden, um zu bestimmen, welche Art von Betriebsdaten in einem späteren Schritt über den ersten Kommunikationskanal mit dem mindestens einen zusätzlichen Gerät kommuniziert werden soll.

In einem dritten Schritt 403 wird ein erster Kommunikationskanal zwischen dem elektrochirurgischen Generator und dem einen oder den mehreren Zusatzgeräten eingerichtet. Daher können die jeweiligen Geräte die in den vorangegangenen Schritten vereinbarten Kommunikationskanalparameter verwenden, um Daten über ihre jeweiligen drahtlosen Kommunikationseinheiten zu senden und zu empfangen.

In einem vierten Schritt 404 übermitteln der elektrochirurgische Generator und das eine oder die mehreren Zusatzgeräte Betriebsdaten über den ersten Kommunikationskanal. Die Betriebsdaten können Aktivierungs- oder Deaktivierungssignale enthalten, die von einem drahtlosen Fußschalter an den elektrochirurgischen Generator oder vom elektrochirurgischen Generator an eine Absaugung oder eine Spülpumpe gesendet werden. Die Betriebsdaten können ferner Soll- oder Ist-Durchflussraten von Pumpen, Soll- oder Ist-Druck der Pumpen oder Ähnliches umfassen. Zu den Betriebsdaten können auch die Soll- oder Ist-Zufluss- und -Abfluss-Temperaturen eines Kühlmittels gehören, das durch eine Kühlmittelpumpe zugeführt werden soll.

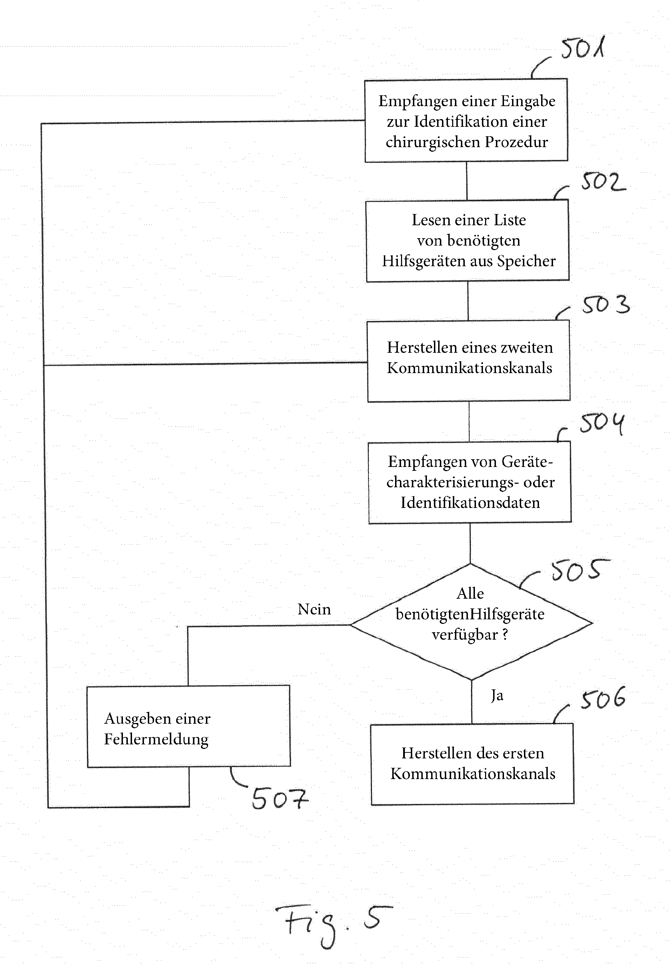

Fig. 5 zeigt ein weiteres Verfahren zum Betrieb eines chirurgischen Gerätesystems, wie es hier beschrieben ist. In einem ersten Schritt 501 kann der elektrochirurgische Generator über eine Benutzerschnittstelle Eingabedaten empfangen, die einen durchzuführenden chirurgischen Eingriff identifizieren. Der Schritt 501 kann das Lesen einer Liste verfügbarer Verfahren aus einem Speicher umfassen, bei dem es sich um einen internen Speicher des elektrochirurgischen Generators oder um einen externen Speicher handeln kann. Die Liste der verfügbaren Verfahren kann dann einem Benutzer über die Benutzerschnittstelle angezeigt werden. Die Liste der verfügbaren Verfahren kann auf verschiedene Weise dargestellt werden. In einem Beispiel kann eine Vielzahl virtueller Auswahltasten auf einem Grafikdisplay angezeigt werden, wobei jede virtuelle Taste einem der verfügbaren Verfahren zugeordnet ist. Der Benutzer kann dann eine der Prozeduren auswählen, indem er die dieser Prozedur zugeordnete virtuelle Schaltfläche aktiviert, z. B. durch Berühren eines Touch-Displays an einer Stelle, an der die virtuelle Schaltfläche angezeigt wird. In einem weiteren Beispiel kann die Liste der verfügbaren Verfahren als eine Liste von Textelementen angezeigt werden, die das jeweilige Verfahren benennen und/oder beschreiben. Der Benutzer kann dann mit Hilfe virtueller oder physischer Bildlauftasten durch die Liste der Textelemente blättern und durch Betätigung einer virtuellen oder physischen Auswahltaste ein Verfahren von Interesse auswählen.

In einem zweiten Schritt 502 wird eine Liste von Zusatzgeräten, die für das ausgewählte Verfahren erforderlich sind, aus einem Speicher gelesen. Bei dem Speicher kann es sich wiederum um einen internen Speicher des elektrochirurgischen Generators oder um einen externen Speicher handeln. Die Liste der erforderlichen Zusatzgeräte kann in einer Datenbank gespeichert sein.

In einem dritten Schritt 503 baut der elektrochirurgische Generator einen zweiten Kommunikationskanal mit einem oder mehreren Zusatzgeräten im Bereich des zweiten Kommunikationskanals auf. Dazu sendet der elektrochirurgische Generator ein Abfragesignal, das von jedem zusätzlichen Gerät innerhalb der Reichweite des Abfragesignals empfangen wird. Jedes zusätzliche Gerät, das das Abfragesignal empfängt, kann ein Antwortsignal senden, um den zweiten Kommunikationskanal aufzubauen.

In Schritt 504 empfängt der elektrochirurgische Generator über den zweiten Kommunikationskanal Gerätecharakterisierungs- und/oder Identifikationsdaten von dem einen oder den mehreren zusätzlichen Geräten.

In Schritt 505 prüft der elektrochirurgische Generator, ob alle erforderlichen Zusatzgeräte verfügbar sind, d. h., ob Gerätecharakterisierungs- und/oder Identifikationsdaten von allen erforderlichen Zusatzgeräten empfangen wurden. Wenn alle erforderlichen Zusatzgeräte verfügbar sind, bauen der elektrochirurgische Generator und das eine oder die mehreren Zusatzgeräte in Schritt 506 einen ersten Kommunikationskanal zur Übermittlung von Betriebsdaten während der Durchführung des ausgewählten Verfahrens auf.

Wenn ein oder mehrere erforderliche Zusatzgeräte keine Gerätecharakterisierungs- und/oder Identifikationsdaten über den zweiten Kommunikationskanal geliefert haben, gibt der elektrochirurgische Generator in Schritt 507 eine Fehlermeldung über eine Benutzerschnittstelle aus. In der Fehlermeldung kann angegeben werden, welche Art von Zusatzgerät nicht verfügbar ist. Die Fehlermeldung kann den Benutzer auffordern, eine geeignete Maßnahme zu wählen. Als eine der möglichen Maßnahmen kann ein Benutzer alle notwendigen Aktionen durchführen, um das eine oder die mehreren fehlenden Zusatzgeräte verfügbar zu machen, z. B. durch Einschalten eines Zusatzgeräts, das versehentlich ausgeschaltet wurde, durch Bringen eines Zusatzgeräts in den Bereich des zweiten Kommunikationskanals, das versehentlich außerhalb dieses Bereichs platziert wurde, oder Ähnliches. Der Benutzer kann dann wählen, das Verfahren mit Schritt 503 fortzusetzen. Als weitere verfügbare Maßnahme kann der Benutzer wählen, das Verfahren mit Schritt 501 erneut zu starten, um ein anderes Verfahren zu wählen. Eine weitere Möglichkeit besteht darin, die Fehlermeldung zu ignorieren und mit Schritt 506 fortzufahren.

In einem modifizierten Verfahren, das in den Zeichnungen nicht dargestellt ist, kann der elektrochirurgische Generator zunächst den zweiten Kommunikationskanal mit einem beliebigen zusätzlichen Gerät innerhalb der Reichweite des zweiten Kommunikationskanals aufbauen und Gerätecharakterisierungs- und/oder Identifikationsdaten von einem beliebigen zusätzlichen Gerät innerhalb der Reichweite des zweiten Kommunikationskanals empfangen. Der elektrochirurgische Generator kann dann aus einem Speicher eine Liste von Prozeduren lesen, die mit dem Satz von Zusatzgeräten, die Gerätecharakterisierungs- und/oder Identifikationsdaten geliefert haben, durchgeführt werden können. Die Liste kann dann über die Benutzerschnittstelle angezeigt werden, so dass ein Benutzer eines der verfügbaren Verfahren zur Ausführung auswählen kann. Beim Lesen der Liste der verfügbaren Verfahren kann der elektrochirurgische Generator eine einschließende Strategie anwenden, bei der alle Verfahren in die Liste aufgenommen werden, die mit dem Satz der verfügbaren Zusatzgeräte durchgeführt werden können, oder eine ausschließende Strategie, bei der nur diejenigen Verfahren in die Liste aufgenommen werden, die alle verfügbaren Zusatzgeräte erfordern.

In den oben beschriebenen Beispielen wurde davon ausgegangen, dass der elektrochirurgische Generator als Hauptgerät des Chirurgiegerätesystems fungiert. In einem modifizierten chirurgischen Gerätesystem kann ein separates Gerät, wie z. B. eine medizinische Gerätesteuerung, als Hauptgerät fungieren, und der elektrochirurgische Generator fungiert als ein weiteres Zusatzgerät des chirurgischen Gerätesystems.

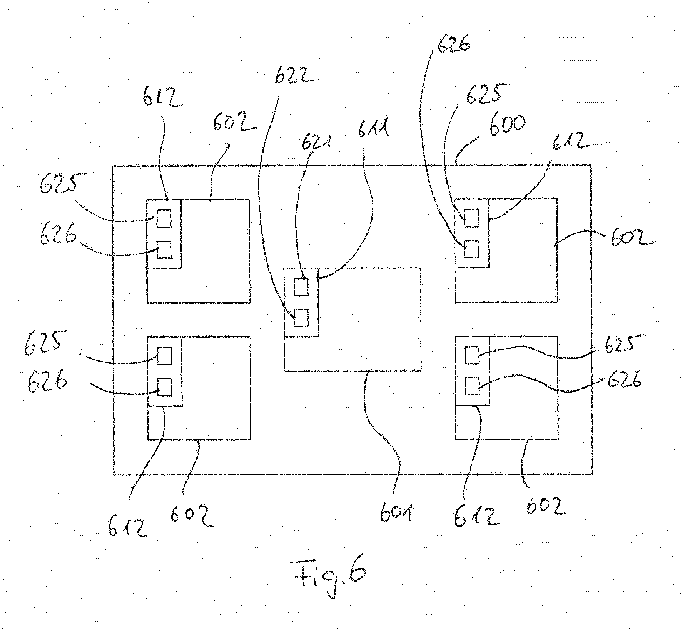

In einem weiteren Beispiel, das in Fig. 6 gezeigt ist, kann ein chirurgisches Gerätesystem ein chirurgisches Gerät 600 in modularer Bauweise umfassen. Das chirurgische Gerät kann eine Master-Einheit 601 und eine oder mehrere Slave-Einheiten 602 umfassen. In Abb. 6 sind vier Slave-Einheiten 602 dargestellt. Es kann auch eine höhere oder niedrigere Anzahl von Slave-Einheiten 602 vorgesehen werden. Die Master-Einheit 601 umfasst eine drahtlose Master-Kommunikationseinheit 611, und jede der Slave-Einheiten 602 kann eine drahtlose Slave-Kommunikationseinheit 612 umfassen. Die drahtlose Hauptkommunikationseinheit 611 kann ein erstes Hauptkommunikationsmerkmal 621 und ein zweites Hauptkommunikationsmerkmal 622 umfassen. Die drahtlose Slave-Kommunikationseinheit 612 kann ein erstes Slave-Kommunikationsmerkmal 625 und ein zweites Slave-Kommunikationsmerkmal 626 umfassen.

Die Master-Einheit 601 kann das zweite Master-Kommunikationsmerkmal 622 verwenden, um einen zweiten Kommunikationskanal zwischen der Master-Einheit 601 und der einen oder den mehreren Slave-Einheiten 602 über die zweiten Slave-Kommunikationsmerkmale aufzubauen. Das zweite Master-Kommunikationsmerkmal 622 kann ein RFID-Abfragesender, ein NFC-Abfragesender oder ähnliches sein. Die zweiten Slave-Kommunikationsmerkmale 626 können RFID-Transponder, NFC-Transponder oder Ähnliches sein.

Zum Aufbau des zweiten Kommunikationskanals kann die Master-Einheit 601 ein Abfragesignal über das zweite Master-Kommunikationsmerkmal 622 senden, und die eine oder mehreren Slave-Einheiten 602 können Antwortsignale über die zweiten Slave-Kommunikationsmerkmale 626 senden.

Nach oder während des Aufbaus des zweiten Kommunikationskanals kommunizieren, verhandeln und/oder vereinbaren die Master-Einheit 601 und die eine oder mehreren Slave-Einheiten 602 Kommunikationskanalparameter für den Aufbau eines ersten Kommunikationskanals unter Verwendung der ersten Master-Kommunikationsfunktion 621 und der einen oder mehreren ersten Master-Kommunikationsfunktionen 625. Das erste Master-Kommunikationsmerkmal 621 und die ersten Slave-Kommunikationsmerkmale 625 können "WiFi"-Antennen, "Bluetooth"-Antennen oder ähnliches sein. Die Master-Einheit 601 und die eine oder mehrere Slave-Einheiten 602 bauen dann den ersten Kommunikationskanal auf, um Betriebsdaten zwischen der Master-Einheit 601 und der einen oder mehreren Slave-Einheiten 602 zu übertragen.

Durch den modularen Aufbau kann das medizinische Gerät 600 flexibel an die spezifischen Bedürfnisse eines Benutzers angepasst werden. Beispielsweise kann das medizinische Gerät 600 ein standardisiertes Rack mit einer Vielzahl von Steckplätzen umfassen, von denen einer von der Master-Einheit 601 und einer Stromversorgungseinheit (nicht dargestellt) belegt werden kann. Die übrigen Steckplätze können für Slave-Einheiten 602 zur Verfügung stehen. Beim Einsetzen einer Slave-Einheit 602 in einen freien Steckplatz kann die Slave-Einheit 602 automatisch mit der Stromversorgungseinheit verbunden werden. Bestimmte Schnittstellen der Slave-Einheiten 602 können an den Vorderseiten der Slave-Einheiten 602 angebracht werden, so dass sie unabhängig davon, wie viele Slave-Einheiten 602 in das Rack eingesetzt werden, zugänglich sind. Zu den Slave-Einheiten 602 können elektrochirurgische Generatoreinheiten, Mikrowellengeneratoreinheiten, Ultraschallgeneratoreinheiten, Spülpumpeneinheiten, Absaugungspumpeneinheiten, Kühlmittelpumpeneinheiten und dergleichen gehören.

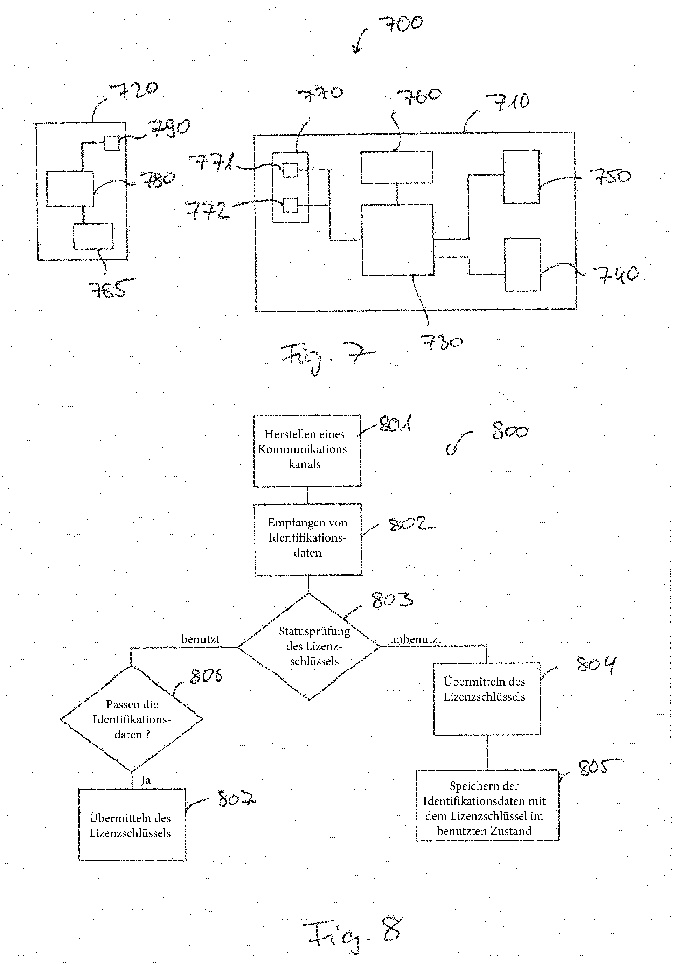

Fig. 7 zeigt ein medizinisches Gerätesystem 700 gemäß einer weiteren Ausführungsform der vorliegenden Offenbarung. Das medizinische Gerätesystem 700 umfasst ein medizinisches Gerät 710 und einen drahtlosen Lizenztag 720.

Das medizinische Gerät kann dem in Fig. 2 gezeigten elektrochirurgischen Generator 10 ähnlich sein und eine Steuereinheit 730, eine Funktionseinheit 740, eine Benutzerschnittstelle 750, eine Speichereinheit 760 und eine drahtlose Kommunikationseinheit 770 mit einem RFID-Abfragesender 771 und einer WiFi-Antenne 772 umfassen.

Das medizinische Gerät 710 ist so eingerichtet, dass es über die Funktionseinheit 740 eine Vielzahl von medizinischen Funktionen bereitstellt. Mindestens eine dieser Funktionalitäten unterliegt einer separaten Lizenz des Herstellers des medizinischen Geräts 710. Um solche Funktionen zu aktivieren, muss eine Lizenz für das medizinische Gerät 710 registriert werden.

Der Benutzer des medizinischen Geräts 710 kann den drahtlosen Lizenztag 720 vom Hersteller des medizinischen Geräts 720 erwerben oder anderweitig beziehen. Der drahtlose Lizenztag 720 umfasst ein Steuergerät 780, ein Speicherelement 785 und eine drahtlose Kommunikationseinheit 790, die ein RFID-Transponder sein kann. Im Speicherelement 785 ist ein Lizenzschlüssel gespeichert.

Ein Verfahren 800 zur Registrierung der Lizenz bei dem medizinischen Gerät 710 ist in Fig. 8 dargestellt.

In einem ersten Schritt 801 wird ein drahtloser Kommunikationskanal zwischen dem medizinischen Gerät 720 und dem drahtlosen Lizenztag 720 aufgebaut. Dazu aktiviert die Steuereinheit 730 den RFID-Interrogator 771, um ein Abfragesignal zu senden, das vom RFID-Transponder 790 des drahtlosen Lizenztags empfangen wird. Der RFID-Transponder 790 sendet ein Antwortsignal an den RFID-Abfragesender 771, wodurch der drahtlose Kommunikationskanal aufgebaut wird.

In einem zweiten Schritt 802 aktiviert die Steuereinheit 730 den RFID-Abfragesender 771, um Identifikationsdaten, die das medizinische Gerät 710 identifizieren, über den drahtlosen Kommunikationskanal zu senden, wobei diese Identifikationsdaten von dem RFID-Transponder 790 empfangen und an die Steuereinheit 780 des drahtlosen Lizenztags weitergeleitet werden.

In einem dritten Schritt 803 überprüft die Steuereinheit 780 des drahtlosen Lizenztags 720 den Status des im Speicherelement 785 gespeicherten Lizenzschlüssels. Der Lizenzschlüssel kann in einem ersten Zustand gespeichert sein, der einen unbenutzten Lizenzschlüssel darstellt. Der Lizenzschlüssel wird in der Regel im ersten Zustand gespeichert, wenn der drahtlose Lizenztag 720 erstmals vom Hersteller des medizinischen Geräts 710 bereitgestellt wird. Der unbenutzte Zustand des Lizenzschlüssels zeigt an, dass der Lizenzschlüssel bei jedem kompatiblen medizinischen Gerät, wie dem medizinischen Gerät 710, registriert werden kann.

Wenn sich der Lizenzschlüssel im ersten Zustand befindet, überträgt die Steuereinheit 780 des drahtlosen Lizenztags 720 den Lizenzschlüssel in Schritt 804 über den drahtlosen Kommunikationskanal an das medizinische Gerät 710.

Um zu verhindern, dass der Lizenzschlüssel mit anderen medizinischen Geräten verwendet wird, nachdem er mit dem medizinischen Gerät 710 verwendet wurde, speichert die Steuereinheit in Schritt 805 den Lizenzschlüssel auf dem Speicherelement 785 in einem zweiten Zustand, der anzeigt, dass der Lizenzschlüssel bereits mit einem bestimmten medizinischen Gerät verwendet wurde. Dazu wird der Lizenzschlüssel zusammen mit den Identifikationsdaten des Medizingeräts 710 auf dem Speicherelement 785 gespeichert. Der Lizenzschlüssel und die Identifikationsdaten können als getrennte Datenelemente gespeichert werden. Der Lizenzschlüssel und die Identifikationsdaten können mittels einer kryptographischen Funktion zu einem einzigen Datenelement zusammengefasst werden. Wenn die Steuereinheit in Schritt 803 feststellt, dass sich der Lizenzschlüssel im zweiten Zustand befindet, wird in Schritt 806 geprüft, ob die in Schritt 802 empfangenen Identifikationsdaten mit den zum Lizenzschlüssel gespeicherten Identifikationsdaten übereinstimmen. Dazu kann ein einfacher Vergleich oder eine inverse kryptographische Funktion angewendet werden.

Wenn die Identifikationsdaten übereinstimmen, wird der Lizenzschlüssel in Schritt 807 erneut an das medizinische Gerät 710 übertragen. Stimmen die Identifikationsdaten nicht überein, wird der Lizenzschlüssel nicht übertragen.

Mit dem beschriebenen Verfahren kann eine Mehrfachverwendung des drahtlosen Lizenztags 720 mit unterschiedlichen medizinischen Geräten verhindert werden, während eine Wiederverwendung des drahtlosen Lizenztags mit demselben medizinischen Gerät möglich ist. Dies kann z.B. notwendig sein, wenn ein am medizinischen Gerät 710 registrierter Lizenzschlüssel verloren geht, z.B. durch einen Hardware-Reset oder ähnliches.

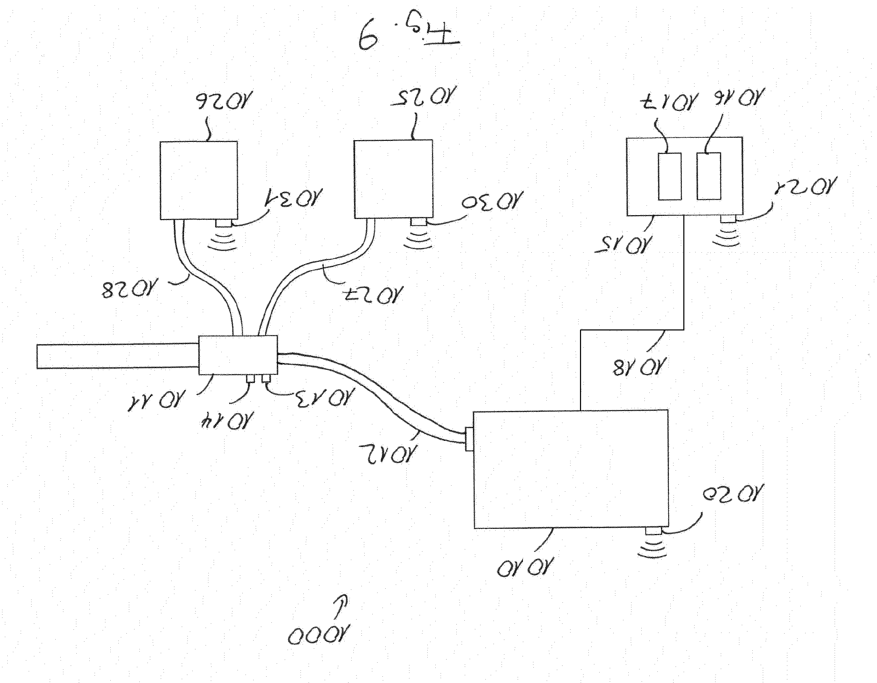

Fig. 9 zeigt ein chirurgisches Gerätesystem 1000 gemäß einer weiteren Ausführungsform der vorliegenden Offenbarung. Das Medizingerätesystem 1000 ähnelt dem in Fig. 1 gezeigten Medizingerätesystem 1, und korrespondierende Elemente sind durch eine um 1000 erhöhte Bezugszahl angezeigt. In der vorliegenden Ausführungsform ist die Fußschaltervorrichtung 1015 mit dem elektrochirurgischen Generator 1010 über eine Kabelverbindung 1018 verbunden.

Während des Betriebs des medizinischen Gerätesystems 1000 kommunizieren der elektrochirurgische Generator 1010 und die Fußschaltervorrichtung 1015 Betriebsdaten über einen ersten, drahtgebundenen Kommunikationskanal, der durch das Kabel 1018 hergestellt wird, und der elektrochirurgische Generator 1010 und die Fußschaltervorrichtung 1015 kommunizieren außerdem zusätzliche Daten über einen zweiten, drahtlosen Kommunikationskanal, der durch die drahtlosen Kommunikationseinheiten 1020, 1021 hergestellt wird.

Bei der vorliegenden Ausführungsform können die Betriebsdaten wesentliche Betriebsdaten umfassen. Zu den wesentlichen Betriebsdaten können Betriebsdaten gehören, die für die Patienten- oder Verfahrenssicherheit relevant sind. Zu den wesentlichen Betriebsdaten können Betriebsdaten gehören, die mit geringer Latenzzeit übertragen werden müssen. Zu den wesentlichen Betriebsdaten können Aktivierungs- und/oder Deaktivierungssignale gehören.

Ergänzende Daten können Gerätecharakterisierungsdaten oder Geräteidentifikationsdaten der Fußschaltervorrichtung 1015 zur Verwendung in einem Verfahren, wie es in Bezug auf Fig. 5 beschrieben ist, umfassen. Zu den zusätzlichen Daten können auch Betriebsdaten gehören, die nicht mit geringer Latenzzeit übertragen werden müssen.

In dem medizinischen Gerätesystem 1000 kann die Anzahl der Adern im Kabel 1018 im Vergleich zu der Anzahl der Adern, die in einem Kabel für ein herkömmliches Zusatzgerät erforderlich sind, reduziert werden. Alternativ kann das Datenvolumen, das zwischen dem Zusatzgerät 1015 und dem elektrochirurgischen Generator 1010 übertragen wird, erhöht werden, z. B. durch Bereitstellung eines neuen Zusatzgeräts 1015 mit erweiterten Funktionen, ohne dass ein vorhandenes Kabel 1018 ersetzt werden muss.

### Weitere Ausführungen

Weitere mögliche Ausführungsformen der Erfindung sind in den folgenden Beispielen dargelegt.

Beispiel 1. Chirurgisches Gerätesystem, umfassend:
- mindestens ein chirurgisches Instrument (11),
- einen chirurgischen Generator (10) zum Bereitstellen chirurgischer Energie für mindestens ein chirurgisches Instrument (11), und
- mindestens eine zusätzliche Vorrichtung (15, 25, 26) zur Bereitstellung einer zusätzlichen Funktion für den chirurgischen Generator (10) und/oder das mindestens eine chirurgische Instrument (11);

wobei der chirurgische Generator (10) so eingerichtet ist, dass er drahtlos mit der mindestens einen zusätzlichen Vorrichtung (15, 25, 26) kommuniziert,
wobei der chirurgische Generator (10) und die mindestens eine Zusatzvorrichtung (15, 25, 26) so eingerichtet sind, dass sie einen ersten Kommunikationskanal zum Austausch von Betriebsdaten einrichten, wobei die erste Kommunikation durch eine Anzahl von ersten Kommunikationskanalparametern definiert ist; und
wobei der chirurgische Generator (10) und das mindestens eine zusätzliche Gerät (15, 25, 26) so eingerichtet sind, dass sie einen zweiten Kommunikationskanal zum Austausch der ersten Kommunikationskanalparameter einrichten.

Beispiel 2. Das chirurgische Gerätesystem nach Beispiel 1, wobei eine Reichweite des zweiten Kommunikationskanals kürzer ist als eine Reichweite des ersten Kommunikationskanals.

Beispiel 3. Das System für chirurgische Geräte nach Beispiel 1 oder 2, wobei eine Bandbreite des zweiten Kommunikationskanals geringer ist als eine Bandbreite des ersten Kommunikationskanals.

Beispiel 4. Das chirurgische Vorrichtungssystem nach einem der Beispiele 1 bis 3, wobei der zweite Kommunikationskanal ein Near Field Communication "NFC"-Kanal ist.

Beispiel 5. Das System für chirurgische Geräte nach einem der Beispiele 1 bis 4, wobei der zweite Kommunikationskanal ein Radio Frequency Identification "RFID"-Kanal ist.

Beispiel 6. Das chirurgische Gerätesystem nach einem der Beispiele 1 bis 5, wobei der erste Kommunikationskanal ein IEEE 802.11 "WiFi"-Kommunikationskanal ist.

Beispiel 7. Das chirurgische Gerätesystem nach einem der Beispiele 1 bis 6, wobei der erste Kommunikationskanal ein "Bluetooth"-Kommunikationskanal, ein IEEE802.15.4-Kommunikationskanal oder ein "ZigBee"-Kommunikationskanal ist.

Beispiel 8. Chirurgisches Gerätesystem nach einem der Beispiele 1 bis 7, wobei das mindestens eine zusätzliche Gerät (15, 25, 26) eingerichtet ist, um

Gerätecharakterisierungsdaten und/oder Geräteidentifikationsdaten über den zweiten Kommunikationskanal zu übertragen.

Beispiel 9. Chirurgisches Gerätesystem nach einem der Beispiele 1 bis 8, wobei die mindestens eine zusätzliche Vorrichtung (15, 25, 26) mindestens einen der folgenden Elemente umfasst: einen Fußschalter (15), eine Rauchabsaugung, eine Spülpumpe (25), eine Absaugung (26) und eine Kühlmittelpumpe.

Beispiel 10. Chirurgisches Gerätesystem nach einem der Beispiele 1 bis 9, wobei die Betriebsdaten mindestens eines der folgenden Signale umfassen: ein Aktivierungssignal, ein Deaktivierungssignal, eine Zielflussrate, eine aktuelle Flussrate, einen Zieldruck, einen aktuellen Druck, eine Zieltemperatur, eine aktuelle Temperatur, einen Batteriestatus, eine Verbindungsqualität und ein Herzschlagesignal.

Beispiel 11. Chirurgisches Gerätesystem nach einem der Beispiele 1 bis 10, wobei der elektrochirurgische Generator (10) so eingerichtet ist, dass er über eine Netzwerkverbindung Software-Aktualisierungsdaten für eines oder mehrere der Zusatzgeräte (15, 25, 26) empfängt und solche Software-Aktualisierungsdaten an das eine oder die mehreren Zusatzgeräte (15, 25, 26) übermittelt.

Beispiel 12. Chirurgisches Gerätesystem, umfassend:
- mindestens ein chirurgisches Instrument (1011),
- einen chirurgischen Generator (1010) zur Bereitstellung von chirurgischer Energie für mindestens ein chirurgisches Instrument (1011), und
- mindestens eine zusätzliche Vorrichtung (1015, 1025, 1026) zum Bereitstellen einer zusätzlichen Funktion für mindestens einen der chirurgischen Generatoren (1010) und/oder das mindestens eine chirurgische Instrument (1011);

wobei der chirurgische Generator (1010) so eingerichtet ist, dass er mit der mindestens einen Zusatzvorrichtung (1015, 1025, 1026) kommuniziert,
wobei der chirurgische Generator (1010) und die mindestens eine Zusatzvorrichtung (1015) eingerichtet sind, einen ersten, drahtgebundenen Kommunikationskanal zum Austausch von Betriebsdaten einzurichten; und
wobei der chirurgische Generator (1010) und das mindestens eine Zusatzgerät (1015) so eingerichtet sind, dass sie einen zweiten, drahtlosen Kommunikationskanal zum Austausch von Zusatzdaten einrichten.

Beispiel 13. Chirurgischer Generator eines chirurgischen Gerätesystems nach einem der Beispiele 1 bis 12.

Beispiel 14. Zusatzgerät eines chirurgischen Gerätesystems nach einem der Beispiele 1 bis 12.

Beispiel 15. Verfahren zum Betreiben eines chirurgischen Gerätesystems nach einem der Beispiele 1 bis 11, mit den Schritten:
- Herstellen eines zweiten Kommunikationskanals zwischen dem chirurgischen Generator (10) und dem mindestens einen Zusatzgerät (15, 25, 26),
- Austauschen von ersten Kommunikationskanalparametern über den zweiten Kommunikationskanal,
- Einrichten eines ersten Kommunikationskanals zwischen dem chirurgischen Generator (10) und der mindestens einen zusätzlichen Vorrichtung (15, 25, 26) unter Verwendung der ersten Kommunikationskanalparameter, und
- Austauschen von Betriebsdaten zwischen dem chirurgischen Generator (10) und der mindestens einen Zusatzvorrichtung (15, 25, 26) über den ersten Kommunikationskanal.

Beispiel 16. Verfahren nach Beispiel 15, welches ferner einen Schritt des Übertragens von Gerätecharakterisierungsdaten und/oder Geräteidentifikationsdaten des mindestens einen Zusatzgeräts (1, 25, 26) über den zweiten Kommunikationskanal umfasst.

Beispiel 17. Verfahren zum Betreiben eines chirurgischen Gerätesystems nach einem der Beispiele 1 bis 12, mit den Schritten:
- Empfangen von Eingabedaten, die einen auszuführenden chirurgischen Eingriff identifizieren, über eine Benutzerschnittstelle (120),
- Auslesen einer Liste von Zusatzgeräten, die für den durchzuführenden chirurgischen Eingriff erforderlich sind, aus einem Speicher (130),
- Aufbau eines zweiten Kommunikationskanals zwischen dem elektrochirurgischen Generator (10) und dem einen oder den mehreren Zusatzgeräten (15, 25, 26) innerhalb der Reichweite des zweiten Kommunikationskanals,
- Empfangen von Geräteidentifikationsdaten und/oder Gerätecharakterisierungsdaten über den zweiten Kommunikationskanal von zusätzlichen Geräten (15, 25, 26) innerhalb der Reichweite des zweiten Kommunikationskanals,
- Prüfen, ob alle für die durchzuführende Prozedur erforderlichen Zusatzgeräte Geräteidentifikationsdaten oder Gerätecharakterisierungsdaten als Antwort auf das Abfragesignal geliefert haben, und
- wenn die Prüfung erfolgreich war, Aufbau eines oder mehrerer erster Kommunikationskanäle zwischen dem elektrochirurgischen Generator (10) und den zusätzlichen Geräten (15, 25, 26).

Beispiel 18. Verfahren nach Beispiel 17, mit dem weiteren Schritt::
- wenn die Prüfung nicht erfolgreich war, Ausgeben einer Fehlermeldung über die Benutzerschnittstelle (120), die fehlende Zusatzgeräte identifiziert, die für den durchzuführenden Eingriff erforderlich sind.

Beispiel 19. Medizinische Vorrichtung, die eine Steuereinheit (100) und eine Funktionseinheit (110) umfasst, wobei die medizinische Vorrichtung so eingerichtet ist, dass sie über die Funktionseinheit (110) eine Vielzahl medizinischer Funktionalitäten bereitstellt, wobei mindestens eine der Vielzahl von Funktionalitäten einer Lizenz unterliegt,
wobei das medizinische Gerät ferner eine drahtlose Kommunikationseinheit (20) umfasst, die so eingerichtet ist, dass sie einen drahtlosen Kommunikationskanal mit einem drahtlosen Lizenztag einrichtet und einen Lizenzschlüssel für die mindestens eine Funktionalität von dem drahtlosen Lizenztag erhält.

Beispiel 20. Medizinisches Gerät nach Beispiel 19, wobei das medizinische Gerät so eingerichtet ist, dass es nach dem Aufbau des drahtlosen Kommunikationskanals mit dem drahtlosen Lizenztag medizinische Geräteidentifikationsdaten über den drahtlosen Kommunikationskanal übermittelt.

Beispiel 21. Drahtloser Lizenztag, wobei der drahtlose Lizenztag so eingerichtet ist, dass er einen drahtlosen Kommunikationskanal mit einer medizinischen Vorrichtung einrichtet und einen Lizenzschlüssel an die medizinische Vorrichtung übermittelt, wobei der Lizenzschlüssel in einem Speicherelement des drahtlosen Lizenztags gespeichert ist.

Beispiel 22. Drahtloser Lizenztag nach Beispiel 21, wobei der drahtlose Lizenztag so eingerichtet ist, dass er den Referenzschlüssel in einem ersten Zustand und einem zweiten Zustand speichert, wobei der erste Zustand einen unbenutzten Lizenzschlüssel darstellt und der zweite Zustand einen benutzten Lizenzschlüssel darstellt.

Beispiel 23. Drahtloser Lizenztag nach Beispiel 22, wobei der drahtlose Lizenztag so eingerichtet ist, dass er den Zustand des gespeicherten Lizenzschlüssels nach der Übertragung des Lizenzschlüssels an das medizinische Gerät von dem ersten Zustand in den zweiten Zustand ändert.

Beispiel 24. Drahtloser Lizenztag nach Beispiel 23, wobei der drahtlose Lizenztag so eingerichtet ist, dass er nach dem Aufbau des drahtlosen Kommunikationskanals mit dem medizinischen Gerät Identifikationsdaten des medizinischen Geräts über den drahtlosen Kommunikationskanal empfängt und die Identifikationsdaten des medizinischen Geräts zusammen mit dem Lizenzschlüssel in dem zweiten Zustand speichert.

Beispiel 25. Drahtloser Lizenztag nach Beispiel 24, wobei der drahtlose Lizenztag so eingerichtet ist, dass er, wenn der Lizenzschlüssel in dem zweiten Zustand gespeichert ist, den Lizenzschlüssel nur dann an das medizinische Gerät übermittelt, wenn die vom medizinischen Gerät empfangenen Identifikationsdaten des medizinischen Geräts mit den mit dem Lizenzschlüssel gespeicherten Identifikationsdaten des medizinischen Geräts übereinstimmen.

## Patentansprüche

1. Chirurgisches Gerätesystem, umfassend:
- mindestens ein chirurgisches Instrument (11),
- einen chirurgischen Generator (10) zum Bereitstellen chirurgischer Energie für mindestens ein chirurgisches Instrument (11), und
- mindestens eine zusätzliche Vorrichtung (15, 25, 26) zur Bereitstellung einer zusätzlichen Funktion für den chirurgischen Generator (10) und/oder das mindestens eine chirurgische Instrument (11);
wobei der chirurgische Generator (10) so eingerichtet ist, dass er drahtlos mit der mindestens einen zusätzlichen Vorrichtung (15, 25, 26) kommuniziert,
wobei der chirurgische Generator (10) und die mindestens eine Zusatzvorrichtung (15, 25, 26) so eingerichtet sind, dass sie einen ersten Kommunikationskanal zum Austausch von Betriebsdaten einrichten, wobei die erste Kommunikation durch eine Anzahl von ersten Kommunikationskanalparametern definiert ist; und
wobei der chirurgische Generator (10) und das mindestens eine zusätzliche Gerät (15, 25, 26) so eingerichtet sind, dass sie einen zweiten Kommunikationskanal zum Austausch der ersten Kommunikationskanalparameter einrichten.

2. Das chirurgische Gerätesystem nach Anspruch 1, wobei eine Reichweite des zweiten Kommunikationskanals kürzer ist als eine Reichweite des ersten Kommunikationskanals.

3. Das chirurgische Gerätesystem nach Anspruch 1 oder 2, wobei eine Bandbreite des zweiten Kommunikationskanals geringer ist als eine Bandbreite des ersten Kommunikationskanals.

4. Das chirurgische Vorrichtungssystem nach einem der Ansprüche 1 bis 3, wobei
- der zweite Kommunikationskanal ein Near Field Communication "NFC"-Kanal ist, oder
- der zweite Kommunikationskanal ein Radio Frequency Identification "RFID"-Kanal ist.

5. Das chirurgische Gerätesystem nach einem der Ansprüche 1 bis 4, wobei der erste Kommunikationskanal ein IEEE 802.11 "WiFi"-Kommunikationskanal, ein "Bluetooth"-Kommunikationskanal, ein IEEE802.15.4-Kommunikationskanal oder ein "ZigBee"-Kommunikationskanal ist.

6. Chirurgisches Gerätesystem nach einem der Ansprüche 1 bis 5, wobei das mindestens eine zusätzliche Gerät (15, 25, 26) eingerichtet ist, um Gerätecharakterisierungsdaten und/oder Geräteidentifikationsdaten über den zweiten Kommunikationskanal zu übertragen.

7. Chirurgisches Gerätesystem nach einem der Ansprüche 1 bis 6, wobei die mindestens eine zusätzliche Vorrichtung (15, 25, 26) mindestens einen der folgenden Elemente umfasst: einen Fußschalter (15), eine Rauchabsaugung, eine Spülpumpe (25), eine Absaugung (26) und eine Kühlmittelpumpe.

8. Chirurgisches Gerätesystem nach einem der Ansprüche 1 bis 7, wobei die Betriebsdaten mindestens eines der folgenden Signale umfassen: ein Aktivierungssignal, ein Deaktivierungssignal, eine Zielflussrate, eine aktuelle Flussrate, einen Zieldruck, einen aktuellen Druck, eine Zieltemperatur, eine aktuelle Temperatur, einen Batteriestatus, eine Verbindungsqualität und ein Herzschlagesignal.

9. Chirurgisches Gerätesystem nach einem der Ansprüche 1 bis 8, wobei der elektrochirurgische Generator (10) so eingerichtet ist, dass er über eine Netzwerkverbindung Software-Aktualisierungsdaten für eines oder mehrere der Zusatzgeräte (15, 25, 26) empfängt und solche Software-Aktualisierungsdaten an das eine oder die mehreren Zusatzgeräte (15, 25, 26) übermittelt.

10. Chirurgischer Generator eines chirurgischen Gerätesystems nach einem der Ansprüche 1 bis 9.

11. Zusatzgerät eines chirurgischen Gerätesystems nach einem der Ansprüche 1 bis 9.

12. Verfahren zum Betreiben eines chirurgischen Gerätesystems nach einem der Ansprüche 1 bis 9, mit den Schritten:
- Herstellen eines zweiten Kommunikationskanals zwischen dem chirurgischen Generator (10) und dem mindestens einen Zusatzgerät (15, 25, 26),
- Austauschen von ersten Kommunikationskanalparametern über den zweiten Kommunikationskanal,
- Einrichten eines ersten Kommunikationskanals zwischen dem chirurgischen Generator (10) und der mindestens einen zusätzlichen Vorrichtung (15, 25, 26) unter Verwendung der ersten Kommunikationskanalparameter, und
- Austauschen von Betriebsdaten zwischen dem chirurgischen Generator (10) und der mindestens einen Zusatzvorrichtung (15, 25, 26) über den ersten Kommunikationskanal.

13. Verfahren nach Anspruch 12, welches ferner einen Schritt des Übertragens von Gerätecharakterisierungsdaten und/oder Geräteidentifikationsdaten des mindestens einen Zusatzgeräts (1, 25, 26) über den zweiten Kommunikationskanal umfasst.

14. Verfahren zum Betreiben eines chirurgischen Gerätesystems nach einem der Ansprüche 1 bis 9, mit den Schritten:
- Empfangen von Eingabedaten, die einen auszuführenden chirurgischen Eingriff identifizieren, über eine Benutzerschnittstelle (120),
- Auslesen einer Liste von Zusatzgeräten, die für den durchzuführenden chirurgischen Eingriff erforderlich sind, aus einem Speicher (130),
- Aufbau eines zweiten Kommunikationskanals zwischen dem elektrochirurgischen Generator (10) und dem einen oder den mehreren Zusatzgeräten (15, 25, 26) innerhalb der Reichweite des zweiten Kommunikationskanals,
- Empfangen von Geräteidentifikationsdaten und/oder Gerätecharakterisierungsdaten über den zweiten Kommunikationskanal von zusätzlichen Geräten (15, 25, 26) innerhalb der Reichweite des zweiten Kommunikationskanals,
- Prüfen, ob alle für die durchzuführende Prozedur erforderlichen Zusatzgeräte Geräteidentifikationsdaten oder Gerätecharakterisierungsdaten als Antwort auf das Abfragesignal geliefert haben, und
- wenn die Prüfung erfolgreich war, Aufbau eines oder mehrerer erster Kommunikationskanäle zwischen dem elektrochirurgischen Generator (10) und den zusätzlichen Geräten (15, 25, 26).

15. Verfahren nach Anspruch 14, mit dem weiteren Schritt:
- wenn die Prüfung nicht erfolgreich war, Ausgeben einer Fehlermeldung über die Benutzerschnittstelle (120), die fehlende Zusatzgeräte identifiziert, die für den durchzuführenden Eingriff erforderlich sind.
